# EUROPÄISCHE PATENTSCHRIFT

(11) **EP 0 850 611 B1**
(45) Veröffentlichungstag und Bekanntmachung des Hinweises auf die Patenterteilung: **22.03.2000**
(21) Anmeldenummer: 97120199.1
(22) Anmeldetag: 18.11.1997
(51) Int. Cl.: A61F 5/02

(54) **Wirbelsäulenorthese**
Spinal orthosis
Orthèse spinale

(30) Priorität: 23.12.1996 DE 19654256
(43) Veröffentlichungstag der Anmeldung: 01.07.1998
(73) Patentinhaber: BIEDERMANN MOTECH GmbH, 78054 Villingen-Schwenningen (DE)
(72) Erfinder: Biedermann, Lutz, 78048 VS-Villingen (DE); Harms, Jürgen, 76337 Waldbronn (DE)
(74) Vertreter: Prüfer, Lutz H., Dipl.-Phys.

(56) Entgegenhaltungen:
- EP-A- 0 362 049
- DE-A- 3 049 097
- FR-A- 2 659 547
- FR-A- 2 696 639
- US-A- 4 708 130
- US-A- 5 433 697

## Beschreibung

Die Erfindung betrifft eine Wirbelsäulenorthese.

Die bekannten Wirbelsäulenorthesen sind einstückig ausgebildet. Es ist deshalb eine große Anzahl von Modellen vorrätig zu halten, aus denen dann diejenige auszuwählen ist, die dem einzelnen Träger aufgrund seiner anatomischen Gegebenheiten paßt.

In der FR 2 696 639-A1 ist ein Korsett nach dem Oberbegriff des Anspruchs 1 offenbart. Dieses erlaubt jedoch keine individuelle Anpassung an den Träger.

Aufgabe der Erfindung ist es, ein System zu schaffen, welches die Vorratshaltung bei Wirbelsäulenorthesen erleichtert und eine bessere Anpassung an die körperlichen Gegebenheiten des einzelnen ermöglicht.

Die Lösung ist in Patentanspruch 1 gekennzeichnet. Weiterbildungen sind in den Unteransprüchen gekennzeichnet.

Im weiteren erfolgt die Beschreibung eines Ausführungsbeispieles anhand der Figuren. Von den Figuren zeigen:
- Fig. 1: Eine explosionsartige Darstellung der Teile der Wirbelsäulenorthese ohne Innenpolster und
- Fig. 2: eine perspektivische Darstellung der zusammengesetzten Wirbelsäulenorthese.

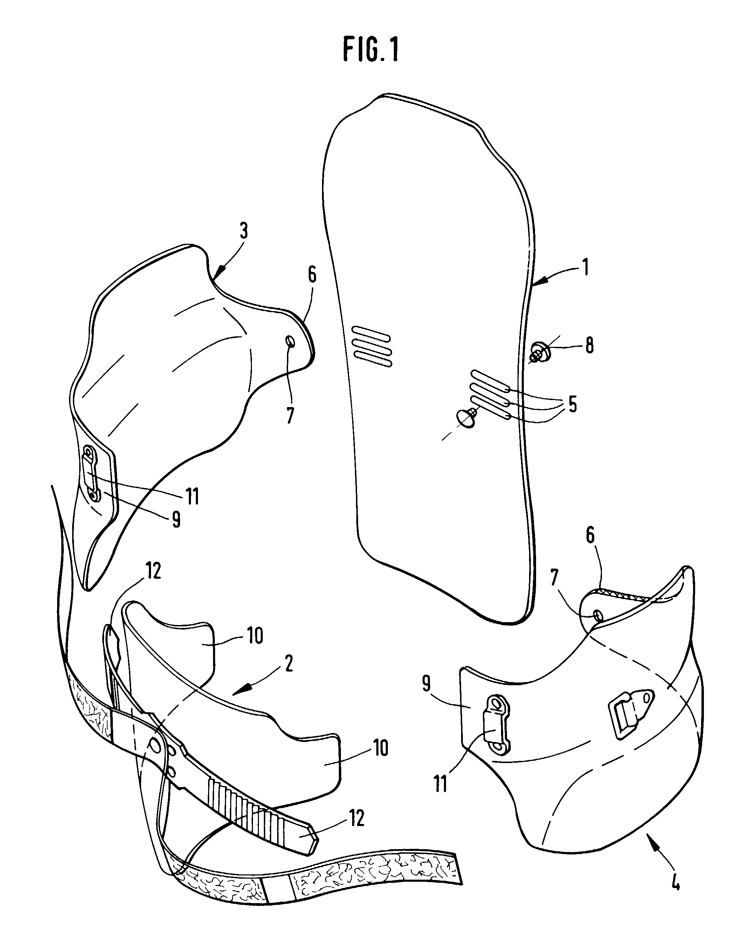

Die Wirbelsäulenorthese weist ein Rückenteil 1, ein Vorderteil 2, ein erstes Seitenteil 3 und ein zweites Seitenteil 4 auf. Jedes der Teile weist eine äußere Hartschale, die jeweils in Fig. 1 gezeigt ist, und vorzugsweise ein inneres Polster wie es in Fig. 1 mit 1', 2', 3' und 4' bezeichnet ist, auf.

Das Rückenteil 1 mit seiner Hartschale und dem Polster 1' ist in seiner Form einem üblichen rückenseitigen Teil einer Wirbelsäulenorthese angepaßt. In dem Bereich, in dem die Seitenteile 3, 4 mit dem Rückenteil zu verbinden sind, sind auf jeder Seite sich in Breitenrichtung erstreckende Langlöcher 5 angeordnet. Ein mittleres Langloch erstreckt sich in Breitenrichtung und ist in einer Höhe angebracht, die einer Normalhöhe der Verbindung zwischen Seitenteil und Rückenteil entspricht. In einem geringen Abstand darüber und darunter sind entsprechende weitere Langlöcher parallel dazu angeordnet.

Die Form der Hartschalen der beiden Seitenteile 3 und 4 und der zugehörigen Polster 3', 4' ist spiegelsymmetrisch ausgebildet. Jedes Teil ist in gleicher Weise wie das Seitenteil der bekannten einstückigen Wirbelsäulenorthese an die Hüftbereiche angepaßt. Die Seitenteile weisen mit dem Rückenteil zu verbindende Scharnierabschnitte 6 auf, die ein Verbindungsloch 7 zur Aufnahme einer Verbindungsschraube 8 aufweisen. Die Verbindung sowohl in der Höhe wie in der Breitenrichtung erfolgt durch Auswahl eines der Langlöcher und der Position in dem ausgewählten Langloch und anschließendes Festziehen der Verbindungsschrauben 8. Die Seitenteile 3, 4 weisen ferner an ihrer der den Scharnierabschnitt 6 aufweisenden Seite abgewandten Seite einen Verbindungsabschnitt 9 auf, der zum Verbinden mit dem Vorderteil 2 dient.

Die in Fig. 1 gezeigte Hartschale des Vorderteiles 2 und das zugehörige Polster 2' sind wie der Bauchbereich bei einer einstückigen Wirbelsäulenorthese an den Bauchbereich angepaßt vorgeformt. Das Vorderteil weist einen zentralen Mittenteil und 2 an den beiden Seiten angebrachte Verbindungsflügel 10 auf, die zum Verbinden mit den Seitenteilen dienen.

Das Mittenteil weist einen in der Mitte mit dem Vorderteil befestigten und auf beiden Seiten freien Gurt 12 auf. An den Verbindungsabschnitten der Seitenteile sind Schnallen 11 vorgesehen, die zur Aufnahme der freien Enden des Gurtes 12 dienen.

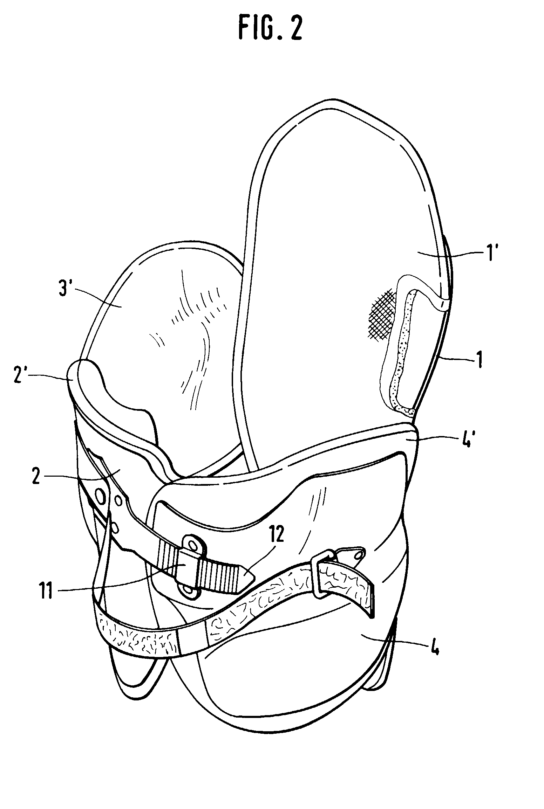

Zum Zusammensetzen umfaßt in der in Fig. 2 gezeigten Weise das den Scharnierabschnitt 6 bildende freie Ende der Seitenteile das Rückenteil von hinten und das die Verbindungsabschnitte 9 bildende jeweilige freie Ende den Verbindungsflügel 10 des Vorderteiles 2 von außen. Nachdem die Höhenlage und Seitenlage der Seitenteile durch die Wahl der Stellung in den Langlöchern 5 bereits gewählt ist, erfolgt die Größenanpassung des Mittenteiles durch entsprechendes Überlagern von Mittenteil und den beiden Seitenteilen und Arretieren mit Hilfe des Gurtes 12.

## Patentansprüche

1. Wirbelsäulenorthese mit
zwei das Becken umfassenden Seitenteilen (3, 4), einem Rückenteil (1) vorbestimmter Höhe und Breite und einem Vorderteil (2) mit einem zentralen Mittenteil wobei
die Seitenteile (3, 4), das Rückenteil (1) und das Vorderteil (2) jeweils eine vorgeformte, äußere Hartschale aufweisen,
dadurch gekennzeichnet, daß das Vorderteil (2) jeweilige seitliche Verbindungsflügel (10) aufweist,
das Rückenteil (1) mit den Seitenteilen (3, 4) jeweils scharnierartig verbunden ist, und
die Seitenteile (3, 4) und das Mittenteil eine Verbindung aus einem Gurt und Schnallen (11) zum einstellbaren Verbinden aufweisen.

2. Wirbelsäulenorthese nach Anspruch 1, dadurch gekennzeichnet, daß das Rückenteil und ein Seitenteil in Breitenrichtung und/oder in Höhenrichtung an verschiedenen Positionen miteinander verbindbar sind.

3. Wirbelsäulenorthese nach Anspruch 2, dadurch gekennzeichnet, daß das Rückenteil und/oder das Seitenteil wenigstens ein sich in Breitenrichtung oder in Höhenrichtung erstreckendes Langloch zur Aufnahme eines Verbindungselementes aufweist.

4. Wirbelsäulenorthese nach einem der Ansprüche 1 bis 3, dadurch gekennzeichnet, daß die Seitenteile mit ihren jeweiligen Rändern außen auf dem Vorderteil und/oder auf dem Rückenteil aufliegen.

## Claims

1. Spinal orthesis having two side parts (3, 4) enclosing the pelvis, a back part (1) of a predetermined height and width and a front part (2) with a central middle part, the side parts (3, 4), the back part (1) and the front part (2) in each case having a preformed, outer hard shell, characterized in that the front part (2) has respective side connecting lugs (10), the back part (1) is connected to the side parts (3, 4) in a hinged manner in each case, and the side parts (3, 4) and the middle part have a connection comprising a belt and buckles (11) for adjustable connecting.

2. Spinal orthesis according to Claim 1, characterized in that the back part and a side part can be connected to each other at various positions in the direction of the width and/or in the direction of the height.

3. Spinal orthesis according to Claim 2, characterized in that the back part and/or the side part has at least one slot extending in the direction of the width or in the direction of the height for receiving a connecting element.

4. Spinal orthesis according to one of Claims 1 to 3, characterized in that the side parts rest with their respective edges on the outside of the front part and/or of the back part.

## Revendications

1. Orthèse pour colonne vertébrale comprenant deux parties latérales (3, 4) entourant le bassin, une partie de dos (1), de hauteur et de largeur prédéfinie, et une partie avant (2) comportant une zone centrale, les parties latérales (3, 4), la partie de dos (1) et la partie avant (2) étant munies chacune d'une coque extérieure rigide pré-formée,
caractérisée en ce que la partie avant (2) comporte sur chaque côté des flancs d'assemblage (10), la partie de dos (1) est assemblée avec chacune des parties latérales (3, 4) à la manière d'un joint mâle et femelle, et les parties latérales (3, 4) et la zone centrale sont assemblées au moyen d'une ceinture et de boucles (11) destinées à former un assemblage réglable.

2. Orthèse pour colonne vertébrale selon la revendication 1, caractérisée en ce que la partie de dos et une partie latérale peuvent être assemblées, dans le sens de la largeur et/ou dans le sens de la hauteur, l'une avec l'autre dans diverses positions.

3. Orthèse pour colonne vertébrale selon la revendication 2, caractérisée en ce que la partie de dos et/ou la partie latérale sont munies d'au moins un trou oblong qui s'étend dans le sens de la largeur ou dans le sens de la hauteur et destiné à recevoir un élément d'assemblage.

4. Orthèse pour colonne vertébrale selon l'une quelconque des revendications 1 à 3, caractérisée en ce que les parties latérales sont posées avec chacun de leurs bords par l'extérieur sur la partie avant et/ou sur la partie de dos.
